# EUROPEAN PATENT SPECIFICATION

(11) **EP 4 171 403 B1**
(45) Date of publication and mention of the grant of the patent: **02.04.2025**
(21) Application number: 21739981.5
(22) Date of filing: 28.06.2021
(51) Int. Cl.: A61B 17/322, A61B 17/3209, A61B 17/00, A61B 17/32

(54) **MULTI-BLADE DERMATOME BLADE ASSEMBLY AND DERMATOME COMPRISING THE SAME**
DERMATOMKLINGENANORDNUNG MIT MEHREREN KLINGEN UND DERMATOM DAMIT
ENSEMBLE LAME DE DERMATOME À LAMES MULTIPLES ET DERMATOME LE COMPRENANT

(30) Priority: 29.06.2020 SE 2050787
(43) Date of publication of application: 03.05.2023
(73) Proprietor: De Soutter Medical Limited, Aylesbury, Bucks HP22 5WF (GB)
(72) Inventor: SJÖBERG, Zacharias, 113 59 Stockholm (SE); SJÖBERG, Folke, 112 35 Stockholm (SE)
(74) Representative: Marks & Clerk LLP
(86) International application number: PCT/EP2021/067695
(87) International publication number: WO 2022/002849

(56) References cited:
- EP-A1- 2 545 869
- DE-A1- 2 939 057
- US-A- 5 951 580
- US-A1- 2009 157 095
- US-A1- 2010 057 100

## Description

### Technical field

The present disclosure relates to the field of dermatomes for surgically harvesting skin grafts. More particularly, the disclosure is directed to dermatome blade assemblies.

### Background art

Dermatomes are devices used for cutting skin tissue to obtain skin grafts for transplantation. Typically, a dermatome has a vibrating or oscillating blade attached to a front end of a main body, which pushes the cutting blade in front of it like a reverse cheese slicer. The oscillation of the blade in a reciprocating side-to-side motion to create a slicing action can be powered either by an electric motor or compressed air. The dermatome currently dominant on the market is of such a design and made by Zimmer Inc., see e.g. EP 2 484 298 A1, US 2013/0018390 A1, US 5,873,881 A1.

Known disclosures of a blade assembly for conventional dermatomes are shown in EP 2 545 869 A1 and US 2009/0157095 A1 which both disclose a single blade mounted on a blade carrier arranged to be secured to a dermatome.

Recent studies have shown that improved results in skin grafting can be achieved by taking multiple thin skin grafts from the same donor site, so-called laminated grafts, instead of the conventional split-thickness of full-thickness skin grafts. According to this technique, the top skin layer (epidermis) is not cut off, but replaced to the original location at the donor site to close the wound after harvesting and allowed to heal again. This leads to substantially reduced healing problems and scarring will be significantly less, i.e. improved results at the donor site.

The second, and possibly third and fourth, layer(s) that is harvested will contain substantially more dermis components, which both provide better mechanical properties at the receiving area, but also contributes more stem cells, which have a higher long-term healing potential as they can divide more times and give rise to new cells than the more superficially located skin cells in the epidermis. This or these layer(s) is/are then used on the injured area. Possibly, the harvested skin layers also contain hair follicles and sebaceous gland cells, which improve the cosmetic appearance of the healed skin long-term and additionally obviates the need for daily rubbing or moisturising the transplanted skin surfaces, as is the case with conventional skin transplants. WO 2017/188888 A1 discloses a novel dermatome comprising multiple blades for simultaneously cutting separate skin grafts at different depths from a donor site.

One problem associated with combining this new technique of cutting multiple skin grafts with conventional dermatomes is that the drive mechanism to oscillate the blade is commonly located in the centre of the head in the form of a drive pin which extends through the blade assembly. As such, there is a risk that the drive pin interferes with or blocks passage of one or more of the cut skin grafts onto respective specially designed collection surfaces of the dermatome.

Hence, there is a need to further develop improved devices and implements for harvesting laminated skin transplants.

### Summary of Invention

An objective of the present disclosure is to provide an improved device which enables simultaneous cutting of separate skin grafts at different depths from a donor site. This objective is achieved by providing a dermatome blade assembly according to the invention as defined in claim 1. The dermatome blade assembly comprising: at least two blades, each including a cutting edge, a rear edge spaced from the cutting edge, and two side edges joining the cutting edge and the rear edge, wherein at least one of the blades comprises at least one throughgoing aperture; and a blade carrier including a main body extending in a transverse direction and means for connecting each of the at least two blades to the blade carrier; wherein the blade carrier comprises a recess arranged to receive a drive pin of a dermatome such that oscillating motion of the drive pin causes reciprocating motion of the dermatome blade assembly.

By means of the dermatome blade assembly, it is possible to connect two or more dermatome blades to the same blade carrier, directly or indirectly. The blade carrier in turn is arranged to be driven by the drive mechanism of a dermatome. Thereby, the reciprocating motion of the blade carrier is conveyed to the at least two blades which enables simultaneous cutting of skin grafts by each of the blades.

In one embodiment, blade connecting means extend from the main body in alignment with the at least one aperture on at least one of the blades, wherein the blade connecting means are arranged to be engaged with said at least one aperture for connecting said least one blade to the blade carrier. The blade connecting means engaged with the aperture provides a simple and reliable connection between the blade and the blade carrier which enables transmitting the transverse reciprocating motion from the blade carrier to the blade.

In one embodiment, the blade connecting means of the blade carrier are in alignment with apertures on each of the at least two blades, wherein the blade connecting means are arranged to be engaged with the respective apertures on each of the at least two blades for connecting each of the at least two blades to the blade carrier. This configuration allows for a direct connection of each of the blades to the blade carrier.

In one embodiment, the at least one blade connecting portion comprises at least one projection arranged to be received in the through-going aperture on each of the at least two blades. The projections allow for a simple, compact and reliable connection between the blades and the blade carrier when the dermatome blade assembly is mounted on a dermatome.

In one embodiment, at least one of the blades is integrally formed with or joined to the blade carrier by means of an adhesive, welding, crimping, moulding, soldering, brazing, or a combination thereof. In this way, a compact, preformed configuration for the connection between the blade and the blade carrier is achieved, requiring fewer assembly steps.

In one embodiment, the dermatome blade assembly further comprises at least one spacer element arranged to be mounted to the dermatome blade assembly in a position between each of the at least two blades to regulate a thickness of a skin graft cut by one of the at least two blades. The spacer element ensures that the thickness of the skin graft cut by lower blade(s) is limited to a desirable thickness instead of taking up all the available space between adjacent blades. Preferably, the dimensions of the spacer element may be varied in accordance with the desired thickness of the skin grafts to be cut. Preferably, the spacer element is adjustable.

In one embodiment, a first, upper blade is arranged offset from a second, lower blade in a cutting direction of the dermatome blade assembly. The offset configuration allows the blades to be brought into action in a staggered manner and enables the operator to maintain the position and inclination of the dermatome during the harvesting operation.

In one embodiment, the recess of the blade carrier is a slot or a through-going aperture arranged in a central position on the main body. The shape of the recess may be adapted to the design of the drive mechanism of the dermatome such that the drive pin is accommodated in the recess in an optimal manner for driving the dermatome blade assembly.

In one embodiment, each of the at least two blades are attached to a bottom side of the blade carrier. This configuration of the blades is adapted to dermatomes of the type having a dedicated space in the head of the main body for accommodating the blade carrier. Dermatomes manufactured by e.g. Nouvag AG are examples of such dermatomes.

In an alternative embodiment, at least a first, upper blade is attached to a top side of the blade carrier and at least a second, lower blade is attached to a bottom side of the blade carrier. This configuration offers a compact solution adapted to dermatomes of the type without a dedicated space in the head of the main body for accommodating the blade carrier. Dermatomes manufactured by e.g. Zimmer Surgical, Inc. are examples of such dermatomes.

In one embodiment, the dermatome blade assembly further comprises at least one intermediate plate, arranged between each of the at least two blades of the dermatome blade assembly and one bottom plate arranged below the lowermost blade of the dermatome blade assembly. The intermediate and bottom plates make up the surrounding structure of the dermatome blade assembly such that only the cutting edges of the two or more blades are exposed. The remaining parts of the blades are protected by the plates whilst allowing for reciprocating motion there within.

In one embodiment, a forward-facing lower surface of the at least one intermediate plate and the bottom plate comprises a central recess, wherein an extension of the recess in the transverse direction is shorter on the bottom plate than on the at least one intermediate plate. By providing a recess of smaller width in the bottom plate, the cutting width of the associated blade is limited such that this blade will not cut into the edges of the groove formed by the preceding blade.

In one embodiment, the recess in the at least one intermediate plate extends from a front end to a rear end of the at least one intermediate plate to form a cavity arranged to allow passage of a skin graft cut by a blade arranged below the at least one intermediate plate. This solution ensures that the cut skin graft by the lower blade(s) can be collected.

In a further aspect of the invention, there is provided a dermatome comprising a dermatome blade assembly according to claim 1.

### Brief Description of Drawings

The invention is now described, by way of example, with reference to the accompanying drawings, in which:
Figs. 1a and 1b show perspective views from below of a dermatome with a dermatome blade assembly according to one embodiment of the present disclosure mounted thereon;
Fig. 2 shows an exploded perspective view of a dermatome with a dermatome blade assembly according to one embodiment of the present disclosure;
Fig. 3 shows an exploded front view of a dermatome with a dermatome blade assembly according to one embodiment of the present disclosure;
Fig. 4a shows an exploded perspective view of a dermatome blade assembly according to one embodiment of the present disclosure;
Fig. 4b shows a perspective view of a dermatome blade assembly according to one embodiment of the present disclosure in an assembled state;
Fig. 5a shows an exploded perspective view of a dermatome blade assembly according to another embodiment of the present disclosure; and
Fig. 5b shows a perspective view of a dermatome blade assembly according to another embodiment of the present disclosure in an assembled state.

### Description of Embodiments

In the following, a detailed description of a dermatome blade assembly according to the present disclosure is presented. In the drawing figures, like reference numerals designate identical or corresponding elements throughout the several figures. It will be appreciated that these figures are for illustration only and do not in any way restrict the scope of the invention.

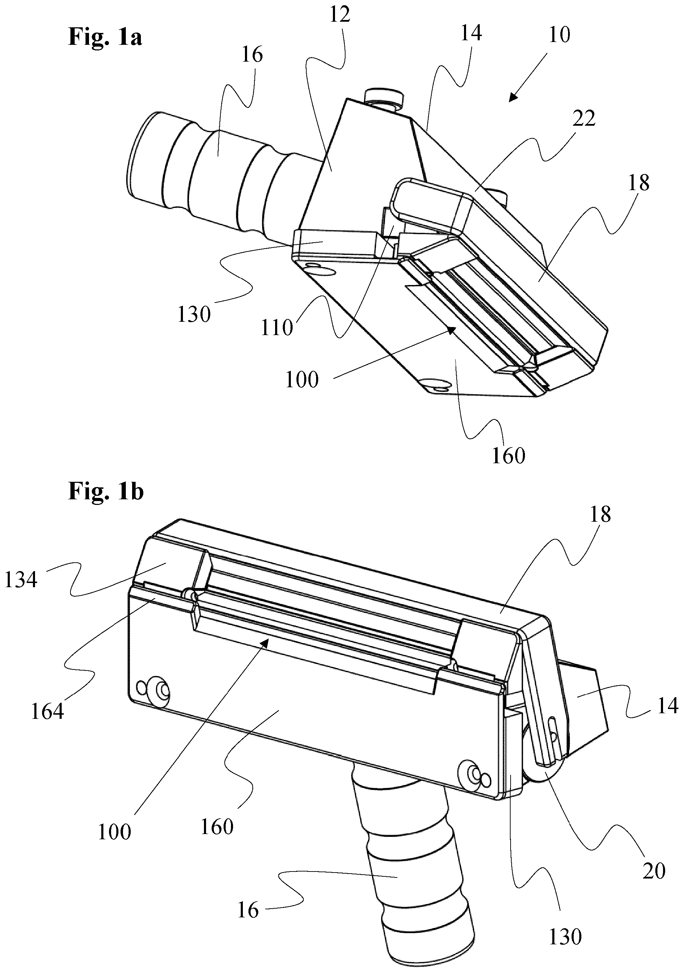

In Figs. 1a and 1b, there is shown an exemplary dermatome 10 comprising a dermatome blade assembly 100 according to one embodiment of the present disclosure mounted thereon. As mentioned above, the dermatome blade assembly 100 may be adapted to the type of dermatome with which it is intended to be used. The dermatome 10 shown in Figs. 1a and 1b should therefore not be construed as limiting in any way, but merely seen as one example, among others.

The dermatome 10 comprises a main body 12 with a head portion 14 connected to a handle 16. The handle 16 may in turn be connected to an external power source (not shown) to oscillate the dermatome blade assembly 100 via a suitable drive mechanism. Alternatively, a motor may be accommodated within the head portion 14 for a standalone dermatome device. Further, the dermatome 10 comprises a thickness regulator in the form of an adjustable bracket 18 mounted on the head portion which may be adjusted by means of a wheel 20 on the side of the head portion. The bracket 18 regulates the thickness of the skin graft through variation of the space between the bracket 18 and a collection surface 22 on the front end of the head portion 14. Typically, the thickness may be regulated in an interval of 0.05-1.0 mm.

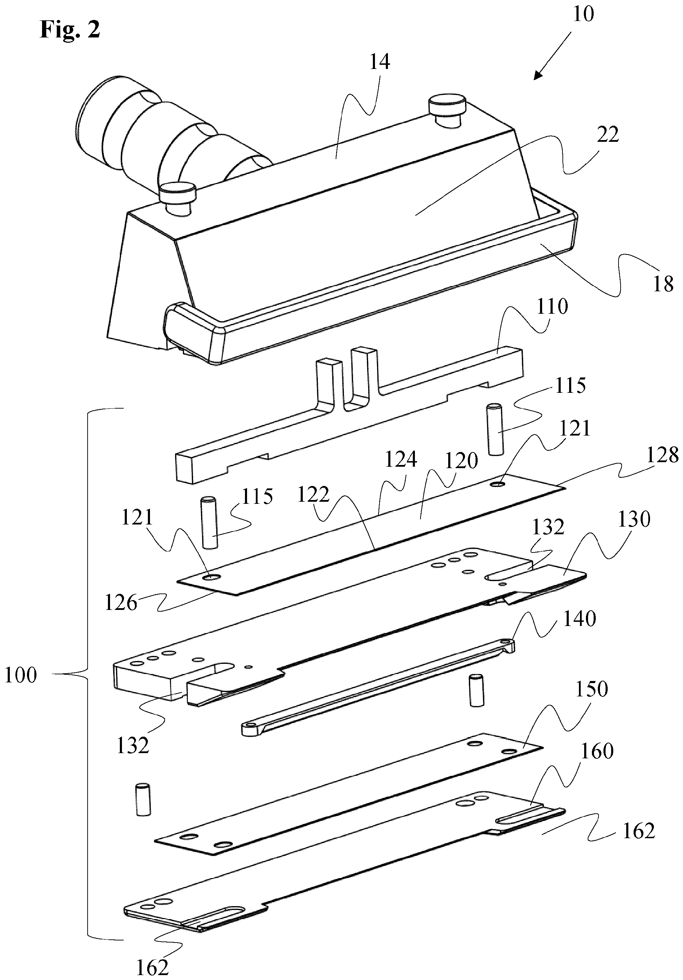

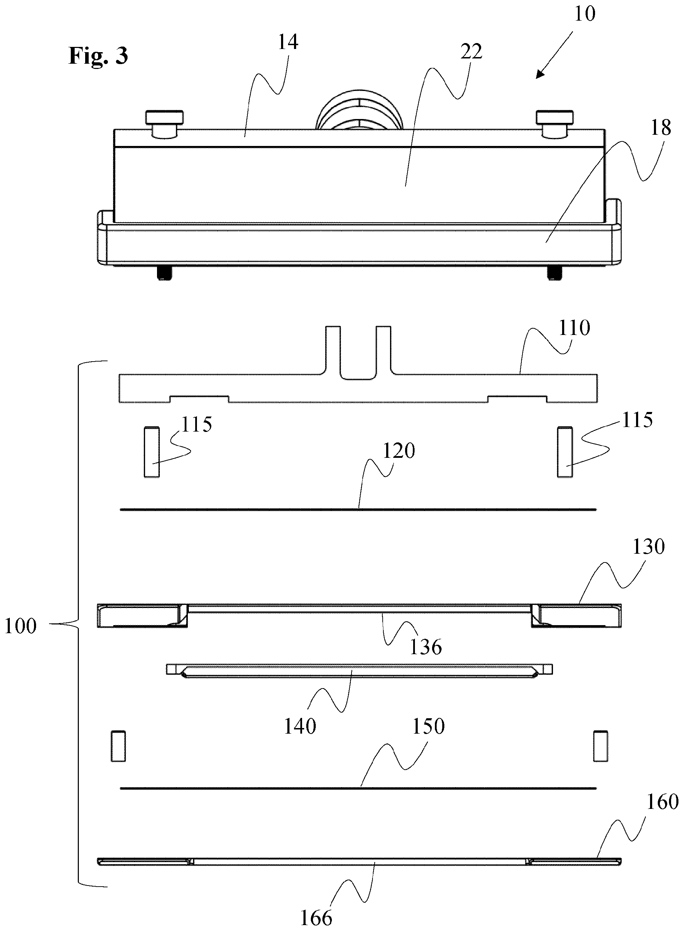

Referring now to Figs. 2 and 3, the dermatome blade assembly 100 according to one embodiment of the present disclosure is shown in an exploded view below the head portion 14 of the dermatome 10 to better illustrate the individual components. From top to bottom in Figs. 2 and 3, there is shown a blade carrier 110, a first blade 120, an intermediate plate 130, a spacer element 140, a second blade 150 and a bottom plate 160. Additionally, a pair of blade connecting means 115 according to one embodiment are shown, to connect the blades 120; 150 to the blade carrier 110.

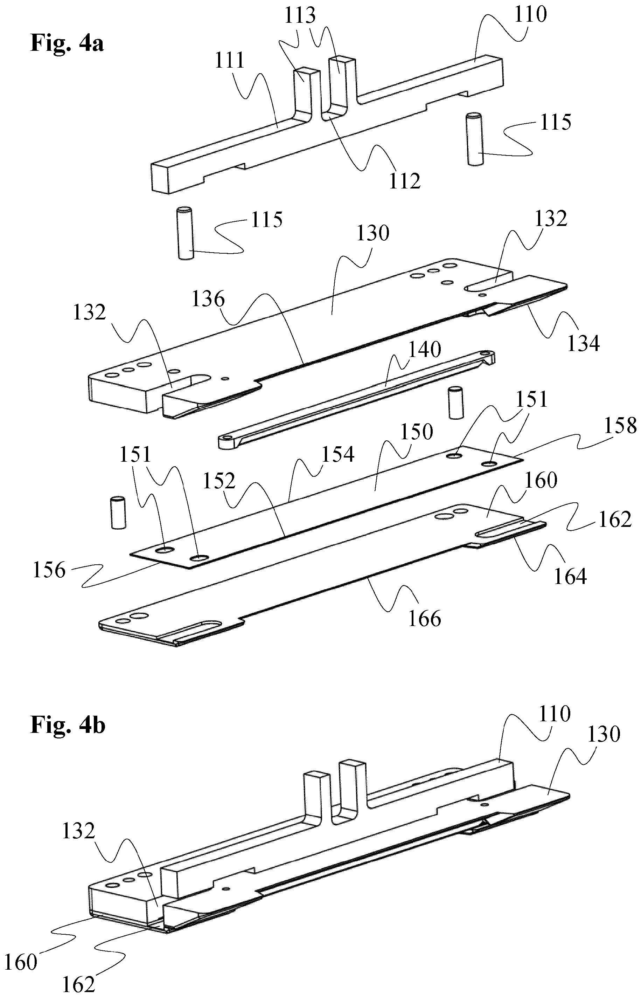

Referring now to Figs. 4a and 4b, blade carrier 110 has a main body 111 which extends in a transverse direction, perpendicular to the cutting direction of the dermatome. On opposite ends of the main body 111, blade connecting means 115 extend downwardly from the main body 111. In one embodiment, the blade connecting means comprises a cylindrical projection 115 which may be formed integrally with or separate from the main body 111. Optionally, the cylindrical projection 115 may be tubular and a pin or screw (not shown) may be inserted through the tubular projection for connecting the blades 120; 150 to the blade carrier 110. The first, upper blade 120 has been omitted in Figs. 4a and 4b for clarity, but should be thought of as being arranged between the blade carrier 110 and the intermediate plate 130 as shown in Figs. 2 and 3. Other suitable cross-sectional shapes of the projections 115 are also encompassed in the present disclosure. Preferably, the blade carrier 110 is manufactured from a suitable plastic material with appropriate characteristics (lightweight, sufficiently rigid) to be used in conjunction with a dermatome. However, other medically acceptable materials such as metals or composites are also encompassed in the present disclosure.

Located centrally on the main body 111, on the side facing away from the blade connecting means 115, there is arranged a recess 112. In one embodiment, the recess 112 is shaped like a slot or fork with two leg portions 113 extending from the main body 111 and arranged to receive a drive pin of the drive mechanism of the dermatome 10. For instance, the drive pin may be arranged eccentrically in relation to a rotation axis such that the rotational motion of the drive pin is translated to a linear reciprocating motion of the blade carrier 110. The main body 111 of the blade carrier 110 is shaped and dimensioned to be accommodated in a space in the head portion 14 of the dermatome 10, as seen in Fig. 1a, to allow for the reciprocating motion of the blade carrier 110.

The blades 120; 150 shown in Figs. 2, 3 and 4a-b are conventional, rectangular metal blades as known in the art with a cutting front edge 122; 152, a rear edge 124; 154 spaced from the cutting edge and two side edges 126, 128; 156, 158 joining the cutting edge 122; 152 and the rear edge 124; 154. The first, upper blade 120 has been omitted in Figs. 3a-3b for improved clarity, but it is understood the dermatome blade assembly includes at least two blades. Other shapes of the blades 120; 150 are also foreseen within the scope of the present disclosure.

Each blade 120; 150 comprises at least one throughgoing aperture 121; 151, which may be located adjacent each of the side edges 126, 128; 156, 158, between the cutting edge 122; 152 and the rear edge 124; 154, so as not to interfere with or block the central portion of the blade 120; 150. However, at least on the first, upper blade 120, other positions along the length of the blade 120 may also be foreseen within the scope of the present disclosure. In the present example, the first blade 120 has one aperture 121 near each side edge 126, 128 whereas the second blade 150 has two apertures 151 adjacent each side edge 156; 158. The additional apertures on the second blade 150 allow for an offset or staggered configuration of the blades 120; 150, as will be further explained below. The apertures 121; 151 may be circular to fit the cylindrical projections 115 of the blade carrier 110. In one embodiment, at least one of the apertures 121; 151 has an oval shape to allow a certain play between the projection 115 and the aperture 121; 151. Other suitable shapes of the apertures 121; 151 are also are also encompassed in the present disclosure.

Alternatively, the first, upper blade 120 may be attached or fastened to the blade carrier 110 using any suitable means, e.g. adhesive, welding, crimping, moulding, soldering, brazing etc. Such a configuration may be preformed to facilitate mounting of the dermatome blade assembly to the dermatome, reducing the number of steps. As an alternative, the blade 120 may be integrally formed with the blade carrier 110.

When attaching or mounting the blades 120; 150 on the blade carrier 110, the blade connecting means 115 are aligned with the apertures 121; 151 on each of the blades 120; 150 and brought into engagement therewith. To this end, there is provided an intermediate plate 130 to be positioned between the two blades 120; 150, and a bottom plate 160 to be positioned beneath the second blade 150. As will be understood, embodiments comprising three or more blades will similarly be provided with two or more intermediate plates, one positioned between adjacent blades of the dermatome blade assembly. The intermediate plate 130 and the bottom plate 160 are fastened to the head portion 14 of the dermatome 10, e.g. by means of screws (not shown), to form a surrounding structure for the dermatome blade assembly 100.

The upper and/or lower surfaces of the intermediate plate 130 and bottom plate 160 facing the respective blades are machined to form indentations in the surface to accommodate the blades 120; 150 and allow the reciprocating motion when the plates 130; 160 are fastened tightly together. The intermediate plate 130 and the bottom plate 160 also comprise lateral fully or partially cut-out portions 132; 162, respectively, aligned with the projections 115 of the blade carrier 110 to allow the reciprocating motion of the blade carrier 110 in relation to the intermediate plate 130 and the bottom plate 160. The forward-facing lower surface 134; 164 of the intermediate plate 130 and the bottom plate 160 is bevelled to provide an inclined working position of the dermatome 10 in order to minimise the area of contact with the skin and reduce friction. To this end, the length from the front to the rear end of the bottom plate 160 is adapted to the end point of the bevelled surface 134 on the intermediate plate 130 to create a smooth transition of the bevelled surfaces 134; 164, as may be seen more clearly in Figs. 1a and 1b.

As mentioned above, an offset configuration of the blades 120; 150 on the blade carrier 110 is used in conjunction with the bevelled surfaces 134; 164 of the intermediate plate 130 and bottom plate 160. To this end, the projections 115 of the blade carrier 110 are aligned with the apertures 121 on the first, upper blade, which are located adjacent the rear edge 124, and the front apertures 151 of the second, lower blade 150, which are located adjacent the cutting edge 152.

Additionally, the central portion of the forward-facing lower surface of each of the intermediate plate 130 and the bottom plate 160 comprises a recess 136; 166 which delimits the cutting width of the dermatome 10. Different intermediate and bottom plates may have recesses 136; 166 of different widths to provide varying cutting widths by exchanging the plates. Preferably, the width of the recess 166 in the bottom plate 160 is smaller than the recess 136 in the intermediate plate 130. This is to ensure that the cutting action of the second, lower blade 150 is limited to a smaller width than the groove cut by the first, upper blade 120, i.e. the second blade will not cut into the edges of the groove. In this way, the second skin graft cut by the second blade 150 will be contained to the dermis layer of the skin to achieve a skin graft with the desired optimal properties as described above.

The recess 136; 166 may be inclined at an angle equal to or different from the bevelled forward-facing lower surfaces 134; 164. On the bottom plate 160, the central recess 166 is limited to the front portion of the lower surface, adjacent the bevelled surfaces 164. However, the skin graft cut by the second, lower blade 150 needs to pass between the intermediate plate 130 and the bottom plate 160. To this end, the central recess 136 in the lower surface of the intermediate plate 130 extends the entire length of the intermediate plate 130, from the front to the rear, to form a cavity which allows passage of the cut skin graft there through. The height of this central recess 136 delimits the available space, and thus, the thickness of the skin graft cut by the second, lower blade 150.

In order to regulate the thickness of the skin graft cut by the second, lower blade 150, there is provided a spacer element 140 arranged to be positioned in the central recess 136 of the intermediate plate 130 when the dermatome blade assembly 100 is mounted on a dermatome 10. The spacer element 140 extends over the full width of the central recess 136 and may be secured to the intermediate plate 130 by means of any suitable fasteners, such as e.g. screws. Different spacer elements 140 may have different shapes and/or dimensions to provide different thicknesses of the cut skin grafts by exchanging the spacer element 140. The forward-facing surface of the spacer element 140 may be shaped to promote passage of the skin graft, e.g. bevelled or rounded.

In one embodiment, the spacer element 140 is adjustable to regulate the available height of the central recess 136. This may be achieved by means of e.g. springs in the mounting between the spacer element 140 and the intermediate plate 130 and an actuating element.

Referring now to Fig. 4b, the dermatome blade assembly 100 is shown in an assembled state, ready to be mounted on the head portion 14 of the dermatome 10. In this view, it may be seen that the cut-out portions 132; 162 form a slot for receiving the projections 115 of the blade carrier 110 extending through the apertures 121; 151 in the blades 120; 150, and allowing the transverse reciprocating motion of the blade carrier 110.

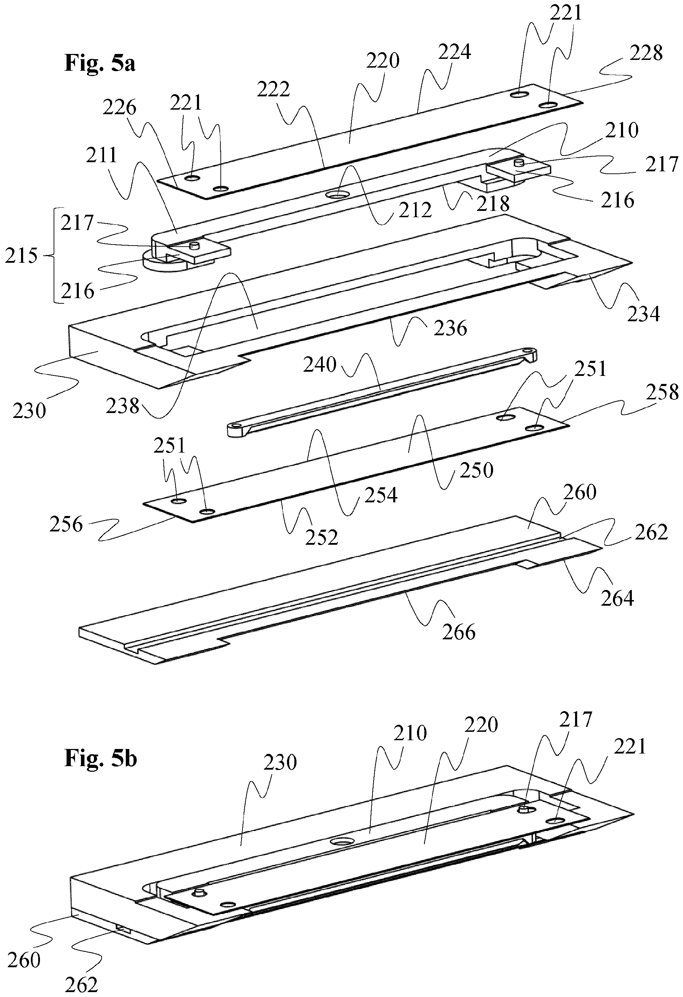

Referring now to Figs. 5a and 5b, there is shown a second embodiment of a dermatome blade assembly 200 according to the present disclosure. Identical or similar features to the ones disclosed in conjunction with the dermatome blade assembly 100 according to the first embodiment, have corresponding reference signs beginning with the numeral "2" instead of "1". Similar to the embodiment illustrated in Figs. 2-5, the dermatome blade assembly 200 comprises a blade carrier 210, a first blade 220, an intermediate plate 230, a spacer element 240, a second blade 250 and a bottom plate 260. The blade carrier 210 differs from the blade carrier 110 in that the blade connecting means 215 comprise ledges 216 on opposite ends of the main body 211 extending in a forward direction. This configuration allows for a more compact dermatome blade assembly 200, suitable to be used with dermatomes which do not have a space for accommodating the blade carrier 210. It is understood that the blade connecting means 215 may extend in a rearward direction as an alternative within the scope of the present disclosure.

On an upward-facing surface of the ledges 216, there is provided a projection 217 which is arranged to be brought into engagement with the apertures 221 in the first, upper blade 220. The upward-facing surface of the ledges 216 may be slightly indented in relation to an upper surface of the main body 211, corresponding to the thickness of the blade 220, such that the upper surface of the blade 220 is substantially flush with the main 211 body of the blade carrier 210 when resting on the ledges 216. As may be seen in Figs. 5a and 5b, the first, upper blade 220 is mounted to the blade carrier 210 from above, whereas the second, lower blade 250 is mounted from below. To this end, blade connecting means in the form of projections (not shown) similar to the projections 217 are provided on a downward-facing side of the blade carrier 210, which engage with the apertures 251 in the second, lower blade 250.

Located centrally on the main body 211 there is arranged a recess 212. In one embodiment, the recess 212 comprises a throughgoing aperture arranged to receive a drive pin of the drive mechanism of the dermatome 20. For instance, the drive pin may be driven in a transverse reciprocating motion in relation to the dermatome, which is then translated to the blade carrier 210 via the throughgoing aperture 212.

The blades 220; 250 shown in Figs. 5a and 5b are similar to the blades 120; 150 discussed in conjunction with Figs. 2, 4a and 4b above. Other shapes of the blades 220; 250 are also foreseen within the scope of the present disclosure. Each blade 220; 250 comprises at least one throughgoing aperture 221; 251 located adjacent each of the side edges 226, 228; 256, 258, between the cutting edge 222; 252 and the rear edge 224; 254. In the present example, the first blade 220 has one aperture 221 near each side edge 226, 228 whereas the second blade 250 has two apertures 251 adjacent each side edge 256; 258. The additional apertures on the second blade 250 allow for an offset or staggered configuration of the blades 220; 250, as will be further explained below. The apertures 221; 251 may be circular to fit the cylindrical projections 217 of the blade carrier 210. In one embodiment, at least one of the apertures 221; 251 has an oval shape to allow a certain play between the projection 215 and the aperture 221; 251. Other suitable shapes of the apertures 221; 251 are also encompassed in the present disclosure.

When attaching or mounting the blades 220; 250 on the blade carrier 210, the blade connecting means 215 are aligned with the apertures 221; 251 on each of the blades 220; 250 and brought into engagement therewith. To this end, there is provided an intermediate plate 230 to be positioned between the two blades 220; 250, and a bottom plate 260 to be positioned beneath the second blade 250. As will be understood, embodiments comprising three or more blades will similarly be provided with two or more intermediate plates, one positioned between adjacent blades of the dermatome blade assembly. The intermediate plate 230 and the bottom plate 260 are fastened to the head portion of a suitable dermatome, e.g. by means of screws (not shown), to form a surrounding structure for the dermatome blade assembly 200.

The upper and/or lower surfaces of the intermediate plate 230 and bottom plate 260 facing the respective blades are machined to form indentations in the surface to accommodate the blades 220; 250 and allow the reciprocating motion when the plates 230; 260 are fastened tightly together. The intermediate plate 230 also comprises a central fully cut-out portion 238, arranged to receive the blade carrier 210 therein and to allow the reciprocating motion of the blade carrier 210 in relation to the intermediate plate 230. As explained above, this embodiment is suitable to be used with dermatomes which do not have a space to accommodate the blade carrier 210.

The bottom plate 260 comprises a transverse groove 262 arranged to receive the downward-facing projections of the blade carrier 210 to allow the reciprocating motion thereof.

The forward-facing lower surface 234; 264 of the intermediate plate 230 and the bottom plate 260 is bevelled to provide an inclined working position of the dermatome in order to minimise the area of contact with the skin and reduce friction. To this end, the length from the front to the rear end of the bottom plate 260 is adapted to the end point of the bevelled surface 234 on the intermediate plate 230 to create a smooth transition of the bevelled surfaces 234; 264.

As mentioned above, an offset configuration of the blades 220; 250 on the blade carrier 210 is used in conjunction with the bevelled surfaces 234; 264 of the intermediate plate 230 and bottom plate 260. To this end, the upper projections 217 of the blade carrier 210 are aligned with the apertures 221 on the first, upper blade, which are located adjacent the rear edge 224, and the lower projections (not shown) engage with the front apertures 251 of the second, lower blade 250, which are located adjacent the cutting edge 252.

In one embodiment, it is foreseen that the blade carrier 210 is directly connected only to the first, upper blade 220 via the upper projections 217. The first blade 220 is then in turn connected to the second blade 250 via separate connecting means through the apertures 221; 251. In this case, the reciprocating motion of the drive pin is transmitted to the blade carrier 210, which drives the first blade 220, which in turn drives the second blade 250.

Additionally, the central portion of the forward-facing lower surface of each of the intermediate plate 230 and the bottom plate 260 comprises a recess 236; 266 which delimits the cutting width of the dermatome 20. Different intermediate and bottom plates may have recesses 236; 266 of different widths to provide varying cutting widths by exchanging the plates. Preferably, the width of the recess 266 in the bottom plate 260 is smaller than the recess 236 in the intermediate plate 230. This is to ensure that the cutting action of the second, lower blade 250 is limited to a smaller width than the groove cut by the first, upper blade 220, i.e. the second blade will not cut into the edges of the groove. In this way, the second skin graft cut by the second blade 250 will be contained to the dermis layer of the skin to achieve a skin graft with the desired optimal properties as described above.

The recess 236; 266 may be inclined at an angle equal to or different from the bevelled forward-facing lower surfaces 234; 264. On the bottom plate 260, the central recess 266 is limited to the front portion of the lower surface, adjacent the bevelled surfaces 264. However, the skin graft cut by the second, lower blade 250 needs to pass between the intermediate plate 230 and the bottom plate 260. To this end, the central recess 236 in the lower surface of the intermediate plate 230 extends the entire length of the intermediate plate 230, from the front to the rear, to form a cavity which allows passage of the cut skin graft there through. The height of this central recess 236 delimits the available space, and thus, the thickness of the skin graft cut by the second, lower blade 250.

In order to regulate the thickness of the skin graft cut by the second, lower blade 250, there is provided a spacer element 240 arranged to be positioned in the central recess 236 of the intermediate plate 230 when the dermatome blade assembly 200 is mounted on a dermatome 20. The spacer element 240 extends over the full width of the central recess 236 and may be secured to the intermediate plate 230 by means of any suitable fasteners, such as e.g. screws. Different spacer elements 240 may have different shapes and/or dimensions to provide different thicknesses of the cut skin grafts by exchanging the spacer element 240. The forward-facing surface of the spacer element 240 may be shaped to promote passage of the skin graft, e.g. bevelled or rounded.

In one embodiment, the spacer element 240 is adjustable to regulate the available height of the central recess 236. This may be achieved by means of e.g. springs in the mounting between the spacer element 240 and the intermediate plate 230 and an actuating element.

Referring now to Fig. 5b, the dermatome blade assembly 200 is shown in an assembled state, ready to be mounted on the head portion of a suitable dermatome.

Preferred embodiments of a dermatome blade assembly have been disclosed above. However, a person skilled in the art realises that this can be varied within the scope of the appended claims.

All the described alternative embodiments above or parts of an embodiment can be freely combined or employed separately from each other as long as the combination is not contradictory.

## Claims

1. A dermatome blade assembly (100; 200), comprising:
at least two blades (120, 150; 220, 250), each including a cutting edge (122, 152; 222, 252), a rear edge (124, 154; 224, 254) spaced from the cutting edge, and two side edges (126, 128, 156, 158; 226, 228, 256, 258) joining the cutting edge and the rear edge, wherein at least one of the blades comprises at least one throughgoing aperture (121, 151; 221, 251); and
a blade carrier (110; 210) including a main body (111; 211) extending in a transverse direction and means for connecting each of the at least two blades to the blade carrier;
wherein the blade carrier comprises a recess (112; 212) arranged to receive a drive pin of a dermatome such that oscillating motion of the drive pin causes reciprocating motion of the dermatome blade assembly.

2. The dermatome blade assembly (100; 200) according to claim 1, wherein blade connecting means (115; 215) extend from the main body in alignment with the at least one aperture on at least one of the blades, wherein the blade connecting means are arranged to be engaged with said at least one aperture for connecting said at least one blade to the blade carrier.

3. The dermatome blade assembly (100; 200) according to claim 2, wherein the blade connecting means (115; 215) of the blade carrier are in alignment with apertures on each of the at least two blades, wherein the blade connecting means are arranged to be engaged with the respective apertures (121, 151; 221, 251) on each of the at least two blades for connecting each of the at least two blades to the blade carrier.

4. The dermatome blade assembly (100; 200) according to claim 2 or 3, wherein the blade connecting means comprise at least one projection (115; 217) arranged to be received in the through-going aperture on at least one of the blades.

5. The dermatome blade assembly (100; 200) according to claim 1 or 2, wherein at least one of the blades is integrally formed with or joined to the blade carrier by means of an adhesive, welding, crimping, moulding, soldering, brazing, or a combination thereof.

6. The dermatome blade assembly (100; 200) according to any one of the preceding claims, further comprising at least one spacer element (140; 240) arranged to be mounted to the dermatome blade assembly in a position between each of the at least two blades to regulate a thickness of a skin graft cut by one of the at least two blades.

7. The dermatome blade assembly (100; 200) according to claim 6, wherein the spacer element is adjustable.

8. The dermatome blade assembly (100; 200) according to any one of the preceding claims, wherein a first, upper blade is arranged offset from a second, lower blade in a cutting direction of the dermatome blade assembly.

9. The dermatome blade assembly (100; 200) according to any one of the preceding claims, wherein the recess (112; 212) of the blade carrier is a slot or a through-going aperture arranged in a central position on the main body.

10. The dermatome blade assembly (100) according to any one of the preceding claims, wherein each of the at least two blades (120; 150) are attached to a bottom side of the blade carrier (110).

11. The dermatome blade assembly (200) according to any one of claims 1-8, wherein at least a first, upper blade (220) is attached to a top side of the blade carrier (210) and at least a second, lower blade (250) is attached to a bottom side of the blade carrier (210).

12. The dermatome blade assembly (100; 200) according to any one of the preceding claims, further comprising at least one intermediate plate (130; 230), arranged between each of the at least two blades of the dermatome blade assembly and one bottom plate (160; 260) arranged below the lowermost blade of the dermatome blade assembly.

13. The dermatome blade assembly (100; 200) according to claim 12, wherein a forward-facing lower surface of the at least one intermediate plate and the bottom plate comprises a central recess (136, 166; 236, 266), wherein an extension of the recess in the transverse direction is shorter on the bottom plate than on the at least one intermediate plate.

14. The dermatome blade assembly (100; 200) according to claim 12 or 13, wherein the recess (136; 236) in the at least one intermediate plate (130; 230) extends from a front end to a rear end of the at least one intermediate plate to form a cavity arranged to allow passage of a skin graft cut by a blade (150; 250) arranged below the at least one intermediate plate.

15. A dermatome (10) comprising a dermatome blade assembly (100; 200) according to any one of the preceding claims.

## Patentansprüche

1. Dermatomklingenanordnung (100; 200), Folgendes umfassend:
mindestens zwei Klingen (120, 150; 220, 250), die jeweils eine Schneidkante (122, 152; 222, 252), eine Hinterkante (124, 154; 224, 254), die von der Schneidkante beabstandet ist, und zwei Seitenkanten (126, 128, 156, 158; 226, 228, 256, 258), welche die Schneidkante und die Hinterkante verbinden, einschließen, wobei mindestens eine der Klingen mindestens eine durchgehende Öffnung (121, 151; 221, 251) umfasst; und
einen Klingenträger (110; 210), der einen Hauptteil (111; 211), der sich in einer Querrichtung erstreckt, und Mittel zum Koppeln jeder der mindestens zwei Klingen mit dem Klingenträger einschließt;
wobei der Klingenträger eine Vertiefung (112; 212) umfasst, die dafür angeordnet ist, einen Antriebsstift eines Dermatoms derart aufzunehmen, dass eine Schwingbewegung des Antriebsstifts eine Hin- und Herbewegung der Dermatomklingenanordnung bewirkt.

2. Dermatomklingenanordnung (100; 200) nach Anspruch 1, wobei sich Klingenkopplungsmittel (115; 215) von dem Hauptteil in Übereinstimmung mit der mindestens einen Öffnung in mindestens einer der Klingen erstrecken, wobei die Klingenkopplungsmittel dafür angeordnet sind, mit der mindestens einen Öffnung in Eingriff zu gelangen, um die mindestens eine Klinge mit dem Klingenträger zu koppeln.

3. Dermatomklingenanordnung (100; 200) nach Anspruch 2, wobei sich die Klingenkopplungsmittel (115; 215) des Klingenträgers in Übereinstimmung mit Öffnungen in jeder der mindestens zwei Klingen befinden, wobei die Klingenkopplungsmittel dafür angeordnet sind, mit den entsprechenden Öffnungen (121, 151; 221, 251) in jeder der mindestens zwei Klingen in Eingriff zu gelangen, um jede der mindestens zwei Klingen mit dem Klingenträger zu koppeln.

4. Dermatomklingenanordnung (100; 200) nach Anspruch 2 oder 3, wobei die Klingenkopplungsmittel mindestens einen Vorsprung (115; 217) umfassen, der dafür angeordnet ist, in der durchgehenden Öffnung in mindestens einer der Klingen aufgenommen zu sein.

5. Dermatomklingenanordnung (100; 200) nach Anspruch 1 oder 2, wobei mindestens eine der Klingen einstückig mit dem Klingenträger gebildet oder mittels eines Haftstoffs, Schweißens, Krimpens, Formpressens, Lötens, Hartlötens oder einer Kombination daraus mit diesem verbunden ist.

6. Dermatomklingenanordnung (100; 200) nach einem der vorhergehenden Ansprüche, ferner mindestens ein Abstandshalterelement (140; 240) umfassend, das dafür angeordnet ist, an einer Position zwischen jeder der mindestens zwei Klingen an der Dermatomklingenanordnung montiert zu sein, um eine Dicke eines Hauttransplantats, das durch eine der mindestens zwei Klingen geschnitten wird, zu regulieren.

7. Dermatomklingenanordnung (100; 200) nach Anspruch 6, wobei das Abstandshalterelement justierbar ist.

8. Dermatomklingenanordnung (100; 200) nach einem der vorhergehenden Ansprüche, wobei eine erste, obere Klinge in einer Schneidrichtung der Dermatomklingenanordnung versetzt zu einer zweiten, unteren Klinge angeordnet ist.

9. Dermatomklingenanordnung (100; 200) nach einem der vorhergehenden Ansprüche, wobei die Vertiefung (112; 212) des Klingenträgers ein Schlitz oder eine durchgehende Öffnung ist, der/die an einer zentralen Position an dem Hauptteil angeordnet ist.

10. Dermatomklingenanordnung (100) nach einem der vorhergehenden Ansprüche, wobei jede der mindestens zwei Klingen (120, 150) an einer Unterseite des Klingenträgers (110) angebracht ist.

11. Dermatomklingenanordnung (200) nach einem der Ansprüche 1 bis 8, wobei mindestens eine erste, obere Klinge (220) an einer Oberseite des Klingenträgers (210) angebracht ist und mindestens eine zweite, untere Klinge (250) an einer Unterseite des Klingenträgers (210) angebracht ist.

12. Dermatomklingenanordnung (100; 200) nach einem der vorhergehenden Ansprüche, ferner umfassend mindestens eine Zwischenplatte (130; 230), die zwischen jeder der mindestens zwei Klingen der Dermatomklingenanordnung angeordnet ist, und eine untere Platte (160; 260), die unter der untersten Klinge der Dermatomklingenanordnung angeordnet ist.

13. Dermatomklingenanordnung (100; 200) nach Anspruch 12, wobei eine nach vorn weisende Unterseite der mindestens einen Zwischenplatte und der unteren Platte eine zentrale Vertiefung (136, 166; 236, 266) aufweist, wobei eine Erstreckung der Vertiefung in der Querrichtung in der unteren Platte kürzer als in der mindestens einen Zwischenplatte ist.

14. Dermatomklingenanordnung (100; 200) nach Anspruch 12 oder 13, wobei sich die Vertiefung (136; 236) in der mindestens einen Zwischenplatte (130; 230) von einem vorderen Ende zu einem hinteren Ende der mindestens einen Zwischenplatte erstreckt, um einen Hohlraum zu bilden, der dafür angeordnet ist, ein Durchtreten eines Hauttransplantats zu ermöglichen, das durch eine Klinge (150; 250) geschnitten wird, die unter der mindestens einen Zwischenplatte angeordnet ist.

15. Dermatom (10), eine Dermatomklingenanordnung (100; 200) nach einem der vorhergehenden Ansprüche umfassend.

## Revendications

1. Ensemble lame de dermatome (100 ; 200) comprenant :
au moins deux lames (120, 150 ; 220, 250), incluant chacune un bord de coupe (122, 152 ; 222, 252), un bord arrière (124, 154 ; 224, 254) espacé du bord de coupe, et deux bords latéraux (126, 128, 156, 158 ; 226, 228, 256, 258) reliant le bord de coupe et le bord arrière, dans lequel au moins une des lames comprend au moins une ouverture traversante (121, 151 ; 221, 251) ; et
un porte-lame (110 ; 210) incluant un corps principal (111 ; 211) s'étendant dans une direction transversale et des moyens pour connecter chacune des au moins deux lames au porte-lame ;
dans lequel le porte-lame comprend un évidement (112; 212) agencé pour recevoir une broche d'entraînement d'un dermatome de sorte qu'un mouvement oscillant de la broche d'entraînement provoque un mouvement de va-et-vient de l'ensemble lame de dermatome.

2. Ensemble lame de dermatome (100 ; 200) selon la revendication 1, dans lequel des moyens de connexion de lame (115 ; 215) s'étendent à partir du corps principal en alignement avec l'au moins une ouverture sur au moins une des lames, dans lequel les moyens de connexion de lame sont agencés pour être en prise avec ladite au moins une ouverture pour connecter ladite au moins une lame au porte-lame.

3. Ensemble lame de dermatome (100 ; 200) selon la revendication 2, dans lequel les moyens de connexion de lame (115 ; 215) du porte-lame sont alignés avec des ouvertures sur chacune des au moins deux lames, dans lequel les moyens de connexion de lame sont agencés pour être en prise avec lesdites ouvertures respectives (121, 151 ; 221, 251) sur chacune des au moins deux lames pour connecter chacune des au moins deux lames au porte-lame.

4. Ensemble lame de dermatome (100 ; 200) selon la revendication 2 ou 3, dans lequel les moyens de connexion de lame comprennent au moins une saillie (115 ; 217) agencée pour être reçue dans l'ouverture traversante sur au moins une des lames.

5. Ensemble lame de dermatome (100 ; 200) selon la revendication 1 ou 2, dans lequel au moins une des lames est formée d'un seul tenant avec ou reliée au porte-lame au moyen d'un adhésif, d'un soudage, d'un sertissage, d'un moulage, d'un brasage ou d'une combinaison de ceux-ci.

6. Ensemble lame de dermatome (100 ; 200) selon l'une quelconque des revendications précédentes, comprenant en outre au moins un élément d'écartement (140 ; 240) agencé pour être monté sur l'ensemble lame de dermatome dans une position entre chacune des au moins deux lames pour réguler une épaisseur d'un greffon de peau prélevé par l'une des au moins deux lames.

7. Ensemble lame de dermatome (100 ; 200) selon la revendication 6, dans lequel l'élément d'écartement est réglable.

8. Ensemble lame de dermatome (100 ; 200) selon l'une quelconque des revendications précédentes, dans lequel une première lame supérieure est agencée de façon décalée par rapport à une deuxième lame inférieure dans une direction de coupe de l'ensemble lame de dermatome.

9. Ensemble lame de dermatome (100 ; 200) selon l'une quelconque des revendications précédentes, dans lequel l'évidement (112 ; 212) du porte-lame est une fente ou une ouverture traversante agencée dans une position centrale sur le corps principal.

10. Ensemble lame de dermatome (100) selon l'une quelconque des revendications précédentes, dans lequel chacune des au moins deux lames (120 ; 150) est fixée à un côté inférieur du porte-lame (110).

11. Ensemble lame de dermatome (200) selon l'une quelconque des revendications 1 à 8, dans lequel au moins une première lame supérieure (220) est fixée à un côté supérieur du porte-lame (210) et au moins une deuxième lame inférieure (250) est fixée à un côté inférieur du porte-lame (210).

12. Ensemble lame de dermatome (100 ; 200) selon l'une quelconque des revendications précédentes, comprenant en outre au moins une plaque intermédiaire (130 ; 230) agencée entre chacune des au moins deux lames de l'ensemble lame de dermatome et une plaque inférieure (160 ; 260) agencée sous la lame inférieure de l'ensemble lame de dermatome.

13. Ensemble lame de dermatome (100 ; 200) selon la revendication 12, dans lequel une surface inférieure orientée vers l'avant de l'au moins une plaque intermédiaire et de la plaque inférieure comprend un évidement central (136, 166 ; 236, 266), dans lequel une extension de l'évidement dans la direction transversale est plus courte sur la plaque inférieure que sur l'au moins une plaque intermédiaire.

14. Ensemble lame de dermatome (100 ; 200) selon la revendication 12 ou 13, dans lequel l'évidement (136 ; 236) dans l'au moins une plaque intermédiaire (130 ; 230) s'étend à partir d'une extrémité avant jusqu'à une extrémité arrière de l'au moins une plaque intermédiaire pour former une cavité agencée pour permettre le passage d'un greffon de peau prélevé par une lame (150 ; 250) agencée sous l'au moins une plaque intermédiaire.

15. Dermatome (10) comprenant un ensemble lame de dermatome (100 ; 200) selon l'une quelconque des revendications précédentes.
